(19) Europäisches Patentamt
European Patent Office
Office européen des brevets

(11) Veröffentlichungsnummer: **0 220 458 A1**

(12) # EUROPÄISCHE PATENTANMELDUNG

(21) Anmeldenummer: **86112756.1**

(22) Anmeldetag: **16.09.86**

(51) Int. Cl.$^4$: **A01N 43/90**, C07D 487/04, //(C07D487/04,249:00,239:00)

(30) Priorität: **28.09.85 DE 3534650**

(43) Veröffentlichungstag der Anmeldung: **06.05.87 Patentblatt 87/19**

(84) Benannte Vertragsstaaten: **AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Jelich, Klaus, Dr.**
**Pahlkestrasse 5**
**D-5600 Wuppertal 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**

(54) **Herbizide Mittel auf Basis von Triazolo-pyrimidinen.**

(57) Die Erfindung betrifft die Verwendung von teilweise bekannten Triazolo-pyrimidinen der Formel (I)

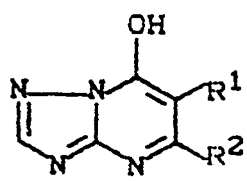

(I)

in welcher
$R^1$ für Wasserstoff oder für Alkyl steht und
$R^2$ für Alkyl steht oder
$R^1$ und $R^2$ für zweifach verknüpftes Alkylen stehen,
wobei jedoch $R^1$ nicht für Wasserstoff steht, wenn gleichzeitig $R^2$ für Methyl steht,
als Herbizide.

EP 0 220 458 A1

Xerox Copy Centre

## Herbizide Mittel auf Basis von Triazolo-pyrimidinen

Die Erfindung betrifft die Verwendung von teilweise bekannten Triazolo-pyrimidinen als Herbizide, insbesondere als selektive Herbizide.

Es ist bereits bekannt, daß bestimmte Triazol-Verbindungen, wie beispielsweise das 3-Amino-1,2,4-(1H)-triazol oder das 5-Methyl-[1,2,4]-triazolo-[1,5a]-pyrimidin-7-ol herbizide Eigenschaften besitzen (vgl. z.B. Französische Patent Anmeldung 1 433 798 sowie K.H. Büchel, "Pflanzenschutz und Schädlingsbekämpfung" S. 180 ff, G. Thieme Verlag, Stuttgart 1977).

Deren herbizide Wirksamkeit gegenüber Problemunkräutern ist jedoch ebenso wie ihre Selektivität gegenüber wichtigen Kulturpflanzen nicht immer in allen Anwendungsbereichen völlig zufriedenstellend.

Es wurde gefunden, daß die teilweise bekannten Triazolopyrimidine der allgemeinen Formel (I),

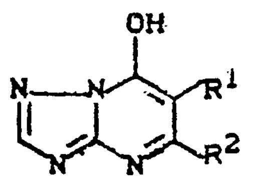

(I)

in welcher

$R^1$ für Wasserstoff oder für Alkyl steht und

$R^2$ für Alkyl steht oder

$R^1$ und $R^2$ für zweifach verknüpftes Alkylen stehen,

wobei jedoch $R^1$ nicht für Wasserstoff steht, wenn gleichzeitig $R^2$ für Methyl steht,

herbizide, insbesondere auch selektiv-herbizide Eigenschaften besitzen.

Dabei zeigt es sich überraschenderweise, daß die erfindungsgemäß verwendbaren Triazolo-pyrimidine der allgemeinen Formel (I) neben einer erheblich besseren Wirksamkeit gegenüber bestimmten Problemunkräutern eine deutlich verbesserte Kulturpflanzenselektivität besitzen als beispielsweise die aus dem Stand der Technik bekannte Verbindung 3-Amino-1,2,4-(1H)-triazol, welches eine chemisch und wirkungsmäßig naheliegende Verbindung ist.

Die erfindungsgemäß verwendbaren Triazolo-pyrimidine sind durch die Formel (I) allgemein definiert. Bevorzugt sind Verbindungen der Formel (I), bei welchen

$R^1$ für Wasserstoff oder für geradkettiges oder verzweigtes Alkyl mit 1 bis 8 Kohlenstoffatomen steht und

$R^2$ für geradkettiges oder verzweigtes Alkyl mit 1 bis 8 Kohlenstoffatomen steht oder

$R^1$ und $R^2$ gemeinsam für einen zweifach verknüpften Alkylenrest mit 3 bis 6 Kohlenstoffatomen stehen, wobei jedoch $R^1$ nicht für Wasserstoff steht, wenn gleichzeitig $R^2$ für Methyl steht.

Besonders bevorzugt sind Verbindungen der Formel (I), bei welchen

$R^1$ für Wasserstoff, Methyl, Ethyl, n-oder i-Propyl sowie n-, i-, s-oder t-Butyl steht und

$R^2$ für Methyl, Ethyl, n-oder i-Propyl sowie n-, i-, s-oder t-Butyl steht oder

$R^1$ und $R^2$ gemeinsam für 1,3-Propandiyl, 1,4-Butandiyl oder1,5-Pentandiyl stehen, wobei jedoch $R^1$ nicht für Wasserstoff steht, wenn gleichzeitig $R^2$ für Methyl steht.

Die erfindungsgemäß verwendbaren Triazolo-pyrimidine sind teilweise bekannt (vgl. z.B. DE-OS 2 247 082 oder US-P-3 526 507).

Noch nicht bekannt sind die folgenden Verbindungen der Formel (I):

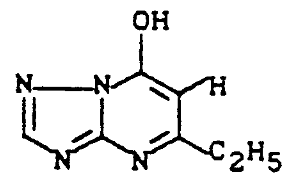
(I-1)

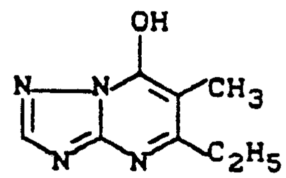
(I-2)

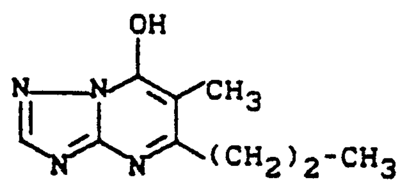
(I-3)

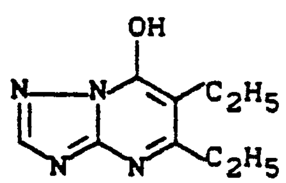
(I-4)

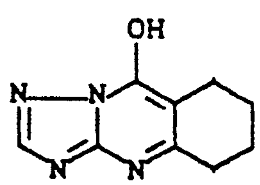
(I-5)

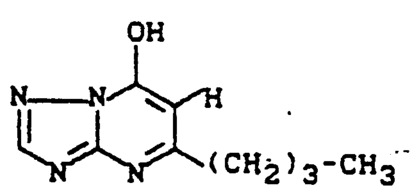
(I-6)

Man erhält die noch nicht bekannten Verbindungen der Formel (I), ebenso wie die bekannten, in Analogie zu bekannten Verfahren (vgl. z.B. Kyushu Diagaku Nogakubu Gakugu Zasski 26, 105-115 [1972] bzw. CA 77: 30142e oder JP 48/31894 vom 2.10.1973), beispielsweise wenn man Aminotriazole der Formel (II),

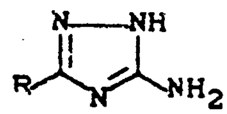
(II)

in welcher
R für Wasserstoff oder für eine Carboxylgruppe steht,
mit β-Ketoestern der Formel (III)

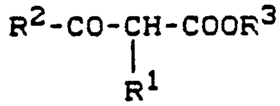 (III)

in welcher
$R^1$ und $R^2$ die oben angegebene Bedeutung haben und
$R^3$ für Alkyl, insbesondere für Methyl oder Ethyl steht,
gegebenenfalls in Gegenwart eines Verdünnungsmittels umsetzt.

Verwendet man beispielsweise 5-Amino-1,2,4-(1H)-triazol-3-yl-carbonsäure und β-Keto-pentansäureethylester als Ausgangsstoffe, so läßt sich der Reaktionsablauf des Herstellungsverfahrens durch das folgende Formelschema darstellen:

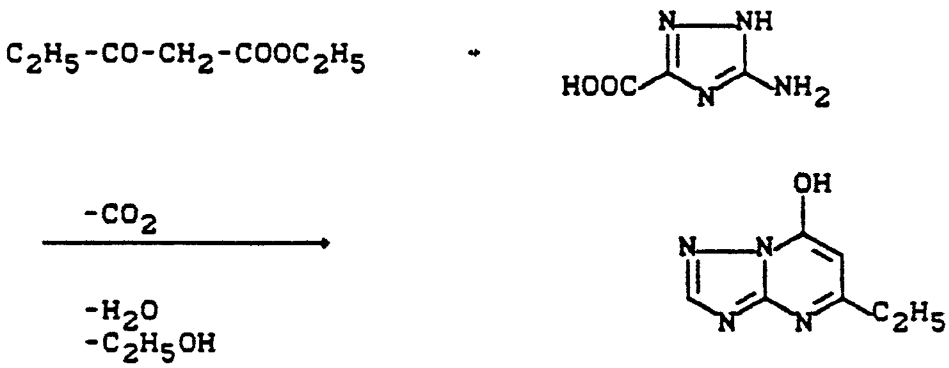


Die zur Durchführung des Herstellungsverfahrens als Ausgangsstoffe benötigten Aminotriazole sind durch die Formel (II) definiert. Die Aminotriazole der Formel (II) sind allgemein bekannte Verbindungen.

Die zur Durchführung des Herstellungsverfahrens weiterhin als Ausgangsstoffe benötigten β-Ketoester sind durch die Formel (III) allgemein definiert. In dieser Formel (III) stehen $R^1$ und $R^2$ vorzugsweise für diejenigen Reste, die im Zusammenhang mit der Beschreibung der erfindungsgemäß verwendbaren Stoffe der Formel (I) als bevor zugt für diese Substituenten genannt wurden. $R^3$ steht vorzugsweise für Methyl oder Ethyl. Die β-Ketoester der Formel (III) sind ebenfalls allgemein bekannte Verbindungen der organischen Chemie.

Als Verdünnungsmittel zur Durchführung des Herstellungsverfahrens kommen inerte organische Lösungsmittel infrage.

Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Alkohole wie Methanol, Ethanol oder Propanol oder Carbonsäuren wie Essigsäure.

Die Reaktionstemperaturen können bei der Durchführung des Herstellungsverfahrens in einem größeren Bereich variiert werden. Im allgemeinen arbeitet man bei Temperaturen zwischen 20 °C und 150°C , vorzugsweise bei Temperaturen zwischen 40 °C und 120 °C.

Zur Durchführung des Herstellungsverfahrens setzt man pro Mol an Aminotriazol der Formel (II) im allgemeinen 1,0 bis 2,5 Mol, vorzugsweise 1,0 bis 1,5 Mol an β-Ketoesterderivat der Formel (III) ein. Die Reaktionsdurchführung, Aufarbeitung und Isolierung der Reaktionsprodukte der Formel (I) erfolgt nach üblichen Verfahren (vg.z.B. J.Chem.Soc.Perkin Trans.I,1980, 1347-1351).

Die erfindungsgemäß verwendbaren Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäß verwendbaren Stoffe als totale oder selektive Herbizide wirken, hängt im wesentlichen von der angewendeten Menge ab.

Die erfindungsgemäß verwendbaren Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäß verwendbaren Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlangen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäß verwendbaren Wirkstoffe mit besonders gutem Erfolg zur selektiven Bekämpfung mono-und dikotyler Umkräuter in mono-und dikotylen Kulturen wie beispielsweise Mais oder Baumwolle einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäß verwendbaren Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H, 3H)-dion oder N-(2-Benzthiazolyl)-N,N'-dimethyl-harnstoff zur Unkrautbekämpfung in Getreide; 4-Amino-3-methyl-6-phenyl-1,2,4,-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit N,N-Dimethyl-N'-(3-trifluormethylphenyl)-harnstoff; 2-[1-(Ethoxamino)-butyliden]-5-(2-ethyl-thiopropyl)-1,3-cyclohexadien; 2-{4-[3-Chlor-5-(trifluormethyl)-2-pyridinyl]-oxy-phenoxy}-propansäure bzw.-propansäureethylester; 2-[4-(3,5-Dichlorpyrid-2-yloxy)-phenoxy]-propionsäure-2-(benzyloxyethylester), -(trimethylsilylmethylester) oder -(2,2-diethoxyethylester); 2-Chlor-4-ethylamino-6-isopropylamino-1,3,5-triazin; 2-Chlor-4-ethylamino-6-(3-cyanopropylamino)-1,3,5-triazin; 2-Chlor-4-ethylamino-6-(3-cyanopropylamino)-1,3,5-triazin; 2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; 6-Chlor-3-phenyl-pyridazin-4-yl-S-octyl-thiocarbonat; 3,5-Diiod-4-hydroxybenzonitril; 3-Isopropyl-2,1,3-benzothiadiazin-4-on-2,2-dioxid; 2-(3,5-Dichlorphenyl)-2-(2,2,2-trichlorethyl)-oxiran;
2-Methoxy-3,6-dichlor-benzoesäure bzw. -benzoesäuremethylester oder3,6-Dichlor-2-pyridincarbonsäure sind gegebenenfalls von Vorteil. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäß verwendbaren Wirkstoffe können sowohl vor als auch nach dem Auflaufen der Pflanzen appliziert werden.
Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäß verwendbaren Wirkstoffe geht aus den nachfolgenden Beispielen hervor.

Herstellungsbeispiele:

Beispiel 1:

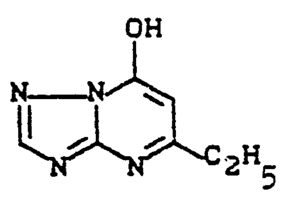

15 g (0,117 Mol) 5-Amino-1,2,4-(1H)-triazol-3-yl-carbonsäure und 20 g ( 0,139 Mol) β-Ketopentansäureethylester werden in 150 ml Eisessig 12 Stunden lang unter Rühren erhitzt, anschließend im Vakuum eingeengt und der Rückstand mit Ether verrührt und abgesaugt.
Man erhält 13,6 g (71 % der Theorie) an 5-Ethyl-s-triazolo-[1,5-a]-pyrimidin-7-ol vom Schmelzpunkt 208° C.

In entsprechender Weise und gemäß den allgemeinen Angaben zur Herstellung erhält man die folgenden Triazolopyrimidine der allgemeinen Formel (I):

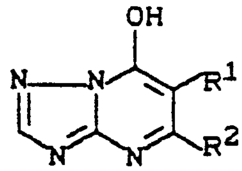

(I)

Tabelle 1

| Bsp. Nr. | $R^1$ | $R^2$ | Schmelz-punkt/° C |
|---|---|---|---|
| 2 | H | $CH_3-(CH_2)_2-$ | 162 |
| 3 | $CH_3$ | $C_2H_5$ | 256 |
| 4 | $C_2H_5$ | $C_2H_5$ | 200 |
| 5 | $-CH_2-CH_2-CH_2-$ | | >270 |
| 6 | $-CH_2-CH_2-CH_2-CH_2-$ | | 262 |
| 7 | $CH_3$ | $CH_3-(CH_2)_3-$ | |
| 8 | $CH_3$ | $CH_3$ | |

Anwendungsbeispiele:

In dem folgenden Anwendungsbeispiel wurde die nachstehend aufgeführte Verbindung als Vergleichsubstanz eingesetzt:

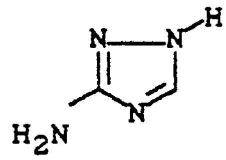

(A)

3-Amino-1,2,4-(1H)-triazol

(bekannt aus K.H. Büchel "Pflanzenschutz und Schädlingsbekämpfungsmittel", G. Thieme Verlag, Stuttgart 1977, S. 181).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 -15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit, ebenso wie in der Nutzpflanzenselektivität, gegenüber dem Stand der Technik zeigen in diesem Test z.B. die Verbindungen gemäß den Herstellungsbeispielen: 1 und 2.

## Ansprüche

1. Herbizide Mittel, gekennzeichnet durch einen Gehalt an mindestens einem Triazolopyrimidin der Formel (I),

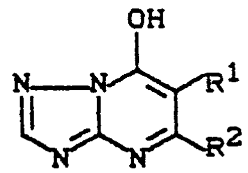

(I)

in welcher
$R^1$ für Wasserstoff oder für Alkyl steht und
$R^2$ für Alkyl steht oder
$R^1$ und $R^2$ für zweifach verknüpftes Alkylen stehen,
wobei jedoch $R^1$ nicht für Wasserstoff steht, wenn gleichzeitig $R^2$ für Methyl steht.

2. Herbizide Mittel, gekennzeichnet durch einen Gehalt an mindestens einem Triazolo-pyrimidin der Formel (I) gemäß Anspruch 1, in welcher
$R^1$ für Wasserstoff oder für geradkettiges oder verzweigtes Alkyl mit 1 bis 8 Kohlenstoffatomen steht und
$R^2$ für geradkettiges oder verzweigtes Alkyl mit 1 bis 8 Kohlenstoffatomen steht oder
$R^1$ und $R^2$ gemeinsam für einen zweifach verknüpften Alkylenrest mit 3 bis 6 Kohlenstoffatomen stehen, wobei jedoch $R^1$ nicht für Wasserstoff steht, wenn gleichzeitig $R^2$ für Methyl steht.

3. Herbizide Mittel, gekennzeichnet durch einen Gehalt an mindestens einem Triazolo-pyrimidin der Formel (I) gemäß Anspruch 1, in welcher
$R^1$ für Wasserstoff, Methyl, Ethyl, n-oder i-Propyl sowie n-, i-, s-oder t-Butyl steht und
$R^2$ für Methyl, Ethyl, n-oder i-Propyl sowie n-, i-, s-oder t-Butyl steht oder
$R^1$ und $R^2$ gemeinsam für 1,3-Propandiyl, 1,4-Butandiyl oder 1,5-Pentandiyl stehen, wobei jedoch $R^1$ nicht für Wasserstoff steht, wenn gleichzeitig $R^2$ für Methyl steht.

4. Verfahren zur Bekämpfung von Unkraut, dadurch gekennzeichnet, daß man Triazolo-pyrimidine der Formel (I) gemäß Anspruch 1 auf Unkraut und/oder deren Lebensraum einwirken läßt.

5. Verwendung von Triazolo-pyrimidinen der Formel (I) gemäß Anspruch 1 zur Bekämpfung von Unkraut.

6. Verfahren zur Herstellung von herbiziden Mitteln, dadurch gekennzeichnet, daß man Triazolo-pyrimidine der Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung

EP 86 11 2756

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | FR-A-1 433 798 (FAIRMOUNT CHEMICAL CO.)<br>----- | 1-6 | A 01 N 43/90<br>C 07 D 487/04 //<br>(C 07 D 487/04<br>C 07 D 249:00<br>C 07 D 239:00 ) |
| | | | **RECHERCHIERTE SACHGEBIETE (Int. Cl.4)** |
| | | | A 01 N |

Der vorliegende Recherchenbericht wurde für alle Patentansprüche erstellt.

| Recherchenort | Abschlußdatum der Recherche | Prüfer |
|---|---|---|
| DEN HAAG | 15-01-1987 | DECORTE D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument